# EUROPÄISCHE PATENTSCHRIFT

(11) **EP 0 589 155 B1**
(45) Veröffentlichungstag und Bekanntmachung des Hinweises auf die Patenterteilung: **06.10.1999**
(21) Anmeldenummer: 93110569.6
(22) Anmeldetag: 02.07.1993
(51) Int. Cl.: C12P 5/02, C12S 3/00

(54) **Anaerobe Behandlung stark fetthaltiger Substanzen**
Anaerobic treatment of substrates with high fat content
Traitement anaérobique de substrats à forte concentration en graisses

(30) Priorität: 08.09.1992 DE 4229986
(43) Veröffentlichungstag der Anmeldung: 30.03.1994
(73) Patentinhaber: ENTSORGUNGS- UND AUFBEREITUNGSGESELLSCHAFT m.b.H., D-08541 Zobes/Vogtl. (DE)
(72) Erfinder: Buschner, Gunter, D-08541 Voigtsgrün/Vogtl. (DE)
(74) Vertreter: Hoeger, Stellrecht & Partner

(56) Entgegenhaltungen:
- WO-A-89/00548
- DATABASE WPI Section Ch, Week 8150, Derwent Publications Ltd., London, GB; Class D16, AN 81-91721D & JP-A-56 139 200 (MATSUSHITA ELEC WORKS) 30. Oktober 1981
- R. BRAUN: "Biogas-Methangärung organischer Abfallstoffe", SPRINGER-VERLAG, WIEN

## Beschreibung

Die Erfindung betrifft ein Verfahren zur Entsorgung von stark fetthaltigen Substanzen, wie insbesondere Maschinenleimleder. Derartige stark fetthaltige, insbesondere im wesentlichen aus Fetten bestehende Substanzen, wie sie z. B. bei Fettabscheidern in Nahrungsmittelverarbeitungsbetrieben anfallen, werden seither auf Deponien abgelagert, was mit hohen Kosten verbunden ist und, obschon es sich hierbei um organische Substanzen handelt, die Umwelt in einem nicht unerheblichen Maße belastet. Infolge von Faul- und Zersetzungsprozessen der auf Deponien abgelagerten vorstehenden Substanzen werden übelriechende Gerüche und Gase freigesetzt, wie sie jedermann von ranzigem Fett her bekannt sein dürften. Die Zersetzung auf der Deponie ist überdies ein langwieriger Prozeß.

Der Erfindung liegt daher die Aufgabe zugrunde, ein Entsorgungsverfahren für stark fetthaltige Substanzen anzugeben, das kostengünstig durchführbar ist und das die Umwelt in geringerem Maße belastet als dies bei der Ablagerung derartiger Substanzen auf Deponien der Fall ist.

Diese Aufgabe wird erfindungsgemäß durch ein Verfahren gelöst, bei dem diese Substanzen unter Beimischung eines ammoniakhaltigen, den PH-Wert stabilisierenden Puffers sowie fettspaltender und Biogas erzeugender Bakterien in einer Biogasanlage anaerob zersetzt werden.

Durch die anaerobe Behandlung und fast vollständige Zersetzung der in Rede stehenden Substanzen wird die aus der Entsorgung resultierende Umweltbelastung erfindungsgemäß auf ein praktisch unerhebliches Maß reduziert. Ein Teil des als Stoffwechselendprodukt anfallenden Biogases ist Methangas; hierdurch eröffnet sich bei dem erfindungsgemäßen Verfahren die Möglichkeit der Energiegewinnung durch die Verbrennung dieses Teils an Luft, also mittels Sauerstoff, zu Kohlendioxid und Wasser. Ein im Zuge der Zersetzung anfallender schlammartiger Rückstand kann als organischer Dünger wiederverwertet werden. Das erfindungsgemäße Entsorgungsverfahren ist daher rückstandslos, d. h. ohne daß nicht weiter verarbeitbare feste Substanzen anfallen, die abgelagert werden müßten, durchführbar.

Es ist zwar seit langem bekannt, Abwässer, Schlämme, organische Abfälle sowie Abprodukte aus der Tierproduktion, insbesondere solche mit hohem Zelluloseanteil, wie Hühnergülle, auf anaerobe Weise in einem Faulbehälter einer Biogasanlage zu zersetzen und das dabei gewonnene Biogas bzw. dessen Methananteil zur Energiegewinnung zu benutzen; man war jedoch der Auffassung, daß sich stark fetthaltige oder im wesentlichen aus Fetten bestehende Substanzen einer anaeroben Zersetzung in einer Biogasanlage entziehen, da bei der Zersetzung großer Mengen derartiger Substanzen enorme Mengen von Fettsäuren entstehen, die zu einer Übersäuerung des im Faulbehälter enthaltenen Mediums führen würden, was ein Absterben der für die Zersetzung und für die Biogas- oder Methanbildung oder -fermentation benötigten Bakterien zur Folge hätte.

Repräsentativ für die bisherige Auffassung der Fachwelt ist das Buch von R. Braun "Biogas-Methangärung organischer Abfallstoffe", Springer-Verlag, Wien, 1982, in dem bezüglich Fettsäuren ausgeführt wird (siehe letzter Absatz der S. 26 sowie Beginn der S. 27), daß normalerweise bei einer Fettsäurekonzentration von 15.000 mg/l die Methanbildung zum Erliegen kommt und unter besonderen Umständen Fettsäurekonzentrationen bis zu 6.000 mg/l keinen nachteiligen Einfluß auf die Gärung haben; beide Grenzwerte liegen aber markant unter denjenigen Fettsäurekonzentrationen, die bei der Verarbeitung von Maschinenleimleder entstehen (würde man Maschinenleimleder ohne Beimischung eines ammoniakhaltigen Puffers anaerob zersetzen wollen, würden bei der Verarbeitung von 1 kg Maschinenleimleder ca. 95.000 mg Fettsäuren entstehen). Bezüglich der Verarbeitung von Gülle in Biogasanlagen wird in diesem Dokument darauf hingewiesen (letzter Absatz der S. 73 und letzter Absatz der S. 75), daß einer beliebigen Erhöhung der Raumbelastung biologische Schwierigkeiten, wie Fettsäure-Toxizität, im Wege stehen, daß Gülle oft von Hause aus einen sehr hohen Fettsäuregehalt besitzt und daß dadurch bei unkontrollierter Gärung sehr rasch eine Überbelastung und ein Absinken des pH-Werts der Gärung unterhalb des üblichen Bereichs von 6,8 bis 8,0 eintreten; dies soll nach diesem Dokument zum Erliegen der Methangärung führen (siehe die beiden letzten Zeilen der S. 73). Schließlich wird noch darauf hingewiesen (siehe die beiden ersten Zeilen des letzten Absatzes der S. 75), daß im Vergleich zu Schweinegülle Hühnermist bei der Methangärung eine Reihe zusätzlicher Probleme verursacht.

Schließlich ergibt sich aus der JP-A-56 139 200 ein Verfahren zum Vergären von Abfall, und zwar offensichtlich von Haushaltsabfall (Gemüse, Fisch und dergleichen), dem ein die Fermentation fördernder Zusatz zugefügt wird, und zwar offensichtlich als quasi Nährlösung für die Mikroorganismen. Dieser Zusatz wird außerhalb des Reaktors gemischt; er enthält zwar auch Fett, wie Butter und Margarine, sowie Ammoniumverbindungen, wie Harnstoff, was aber nicht dazu führen kann, daß sich dieses bekannte Verfahren mit dem erfindungsgemäßen Verfahren vergleichen läßt, welches sich mit der Aufarbeitung von stark fetthaltigen Substanzen befaßt - jeder Fachmann weiß, daß wenn das zu vergärende Substrat überwiegend aus Fett besteht, bei der Fermentation die vorstehend beschriebenen Probleme auftreten, für deren Beseitigung die JP-A-56 139 200 keinen Anhaltspunkt gibt.

Mit der Erfindung wurde nun erkannt, daß sich sowohl für die fettspaltenden, also Säure bildenden Bakterien, als auch für die Methanbakterien günstige Lebensbedingungen dadurch einstellen lassen, daß den stark fetthaltigen, auf anaerobe Weise zu zersetzenden Substanzen ein ammoniakhaltiger, den PH-Wert stabilisierender Puffer zugesetzt wird. Dieser Puffer führt nämlich zur Neutralisierung der im Zuge der Zersetzung gebildeten enormen Säuremengen und verhindert ein Absinken des PH-Werts unter einen die Lebensbedingungen der Methanbakterien begrenzenden Wert. Das erfindungsgemäße Verfahren ermöglicht es also überhaupt erst, im wesentlichen aus stark fetthaltigen Substanzen bestehendes Substrat auf anaerobe Weise zu zersetzen.

Die fettspaltenden Bakterien sowie die Biogas bzw. Methan erzeugenden Bakterien könnten von einem anaeroben Zersetzungsprozeß beliebiger, bekannterweise anaerob zersetzbarer Substanzen stammen; die stark fetthaltigen Substanzen könnten in diesem Fall in einen bekannte Substanzen enthaltenden Faulbehälter eingebracht und gleichzeitig mit diesen oder im Anschluß an deren Zersetzung unter Übernahme der vorhandenen Bakterienstämme zersetzt werden. Es wäre auch denkbar, fettspaltende sowie Biogas erzeugende Bakterien durch Vermischung der fetthaltigen Substanzen mit Klärschlamm oder durch Beimischung von Schweine- oder Rindergülle bereitzustellen.

In Schweine- oder Rindergülle ist zwar Ammoniak enthalten, der als basischer Puffer wirkt, jedoch in einem für die ausreichende Neutralisierung der im Zuge der Zersetzung entstehenden Fettsäuren unzureichenden Maße. Bei der Durchführung des erfindungsgemäßen Verfahrens unter Verwendung der vorgenannten Gülle könnte jedoch zur "Nachschärfung" des in der Gülle enthaltenen basischen Puffers beispielsweise Ammoniakwasser zugegeben werden, um eine Übersäuerung des Mediums zu verhindern.

Nach einer ganz besonders bevorzugten Verfahrensvariante wird den anaerob zu zersetzenden fetthaltigen Substanzen Hühnergülle beigemischt. In der Hühnergülle, die vorteilhafterweise im Zuge der industriemäßigen Hühnerproduktion oder -haltung in großen Mengen anfällt, sind sowohl Ammoniak als auch fettspaltende und Biogas erzeugende Bakterien in einem für die Durchführung des erfindungsgemäßen Verfahrens geeigneten Maße enthalten. Es kann also z. B. durch Mischung von Hühnergülle mit Maschinenleimleder, das sich durch seine schnelle Zersetzbarkeit und damit verbundener enorm hoher Säureproduktion auszeichnet, ein für die anaerobe Zersetzung hervorragend geeignetes Substratgemisch erhalten werden. Es hat sich aber auch als vorteilhaft erwiesen, den fetthaltigen Substanzen aufgemaischten, d. h. mit Flüssigkeit versetzten, Festmist, insbesondere Hühnermist, beizumischen, der bei der Tierhaltung gleichfalls in großen Mengen anfällt.

Die anaerobe Zersetzung von Hühnergülle ist, wie eingangs bereits erwähnt wurde, an sich bekannt; so befaßt sich beispielsweise die Wirtschaftspatentschrift DD 254 377 Al mit der Möglichkeit der Steigerung der Biogasausbeute bei der Zersetzung von organischen Substanzen, insbesondere solcher mit einem hohen Zelluloseanteil, wie z. B. Hühnergülle. Das in dieser Druckschrift beschriebene Verfahren konzentriert sich jedoch auf eine Effektivitätssteigerung des der Methanfermentation vorangehenden Schrittes der Zellulosespaltung, was dadurch erreicht wird, daß der anaerob zu behandelnden Hühnergülle Rindergülle, die in ausreichender Menge zellulosespaltende Bakterien enthält, als Impfmaterial zugesetzt wird. Ein Hinweis auf die Möglichkeit der anaeroben Behandlung von stark fetthaltigen Substanzen läßt sich dieser Druckschrift jedoch nicht entnehmen.

Auch der Wirtschaftspatentschrift DD 260 397 A3, die eine Feststoffsteuerung innerhalb eines Faulbehälters, d. h. die Steuerung der Feststoffkonzentration eines anaerob zu zersetzenden Substratgemisches zum Gegenstand hat, ist weder ein Hinweis auf das der vorliegenden Erfindung zugrundeliegende Problem noch auf dessen Lösung zu entnehmen, es wird lediglich erwähnt, daß es sich um ein Verfahren zur anaeroben Behandlung organisch belasteter Abwässer, Schlämme und Abprodukte, z. B. aus der Industrie, dem kommunalen Bereich sowie den Einrichtungen der industriellen Tierproduktion handle.

Aufgrund der oft schlechten oder schwierigen Handhabbarkeit von stark fetthaltigen Substanzen, insbesondere des aufgrund seiner Faserstruktur mechanisch kaum zerkleinerbaren, in Stücken anfallenden Maschinenleimleders, wird vorgeschlagen, den zu zersetzenden Substanzen zum Herstellen eines pumpfähigen Substratgemisches zum Einbringen in den Faulbehälter ein flüssiges Medium beizugeben. Dadurch kann zugleich ein einer optimalen Prozeßführung förderlicher Trockensubstanzgehalt in dem gebildeten Substratgemisch eingestellt werden. Ein zu hoher Trockensubstanzgehalt, also eine zu hohe Feststoffkonzentration innerhalb des Faulbehälters, behindert nämlich den Stoffaustausch zwischen den Zellen der zu zersetzenden Substanzen und dem umgebenden Medium, während bei einer zu geringen Feststoffkonzentration die Ausbeute bei der anaeroben Zersetzung aufgrund der hierfür benötigten Volumina der Faulbehälter nicht mehr akzeptierbar wäre.

Die zu zersetzenden Substanzen könnten also beispielsweise mit Frischwasser vermischt und anschließend über Leitungen in den Faulbehälter eingebracht werden. Um jedoch stoßartige Änderungen der Prozeßbedingungen innerhalb des Faulbehälters zu verhindern, empfiehlt es sich, dem flüssigen Medium, zuvor den Puffer sowie die fettspaltenden und Methan erzeugenden Bakterien beizugeben, indem beispielsweise eine Aufmaischung aus Festmist und Frischwasser verwendet wird.

Als ganz besonders vorteilhaft hat es sich erwiesen, als den zu zersetzenden Substanzen beizugebendes flüssiges Medium aus einem Faulbehälter entnommenes Faulwasser zu verwenden, wobei unter Faulwasser in einem Faulbehälter enthaltene Flüssigkeit verstanden wird. Vorzugsweise wird beim Entnehmen von Faulwasser aus dem Faulbehälter dessen Trockensubstanzgehalt durch Abtrennen von Feststoffen erniedrigt. Wenn die zu zersetzenden Substanzen mit warmem, aus dem Faulbehälter entnommenem Faulwasser vermischt werden, so braucht das hierbei entstehende Substratgemisch vor Einbringen in den Faulbehälter nicht erst erwärmt zu werden, um unerwünschte, die Prozeßbedingungen störend beeinflussende Temperatursenkungen innerhalb des Faulbehälters zu vermeiden.

Nach einer weiteren bevorzugten Verfahrensvariante besteht das flüssige Medium, das den zu zersetzenden Substanzen beigemischt wird, zumindest zum Teil aus insbesondere frischer Hühnergülle. Ist nämlich für eine vorgebbare Menge einer bestimmten Substanz die zu ihrer Zersetzung erforderliche oder bevorzugte Menge an Hühnergülle bekannt, so erweist es sich im Sinne der kontinuierlichen Aufrechterhaltung günstiger Prozeßbedingungen als vorteilhaft, das Substratgemisch gleich in seiner bevorzugten Zusammensetzung dem Faulbehälter zuzuführen.

Es empfiehlt sich ferner, das pumpfähige Substratgemisch in einen oberen Bereich des Faulbehälters einzubringen, um zu verhindern, daß mit dem hierdurch aus einem unteren Bereich des Faulbehälters über Rohrleitungen verdrängten Faulwasser gerade eingebrachtes Substrat wieder aus dem Faulbehälter herausgeschwemmt wird.

Wenn ein erhöhter Trockensubstanzgehalt im Faulbehälter ein Verdünnen des darin befindlichen Faulwassers mittels Frischwassers oder mittels verdünnter Gülle erforderlich machen, oder wenn dem Faulbehälter zu zersetzende Feststoffe zugeführt werden - seien es stark fetthaltige oder andere Substanzen - und infolgedessen entsprechend der zugeführten Menge Faulwasser aus dem Faulbehälter entnommen werden muß, wird in Weiterbildung der Erfindung vorgeschlagen, das entnommene Faulwasser einer Nachfaulung in einem weiteren, im wesentlichen nur Faulwasser enthaltenden Faulbehälter, zu unterziehen. Hierdurch wird ein noch vollständigerer Abbau erreicht; das Faulwasser aus dem weiteren Faulbehälter kann hiernach z. B. der landwirtschaftlichen Verwertung zugeführt oder zur Kompostbefeuchtung verwendet werden.

Bei der aus dem Stand der Technik bekannten anaeroben Behandlung organischer Substanzen wurde als oberer Grenzwert der Säurekonzentration, oberhalb dessen ein drastischer Rückgang der spezifischen, also auf eine Menge organischer Trockensubstanz bezogenen Gasproduktion zu verzeichnen ist, ein Wert von 2000 - 3000 mg/l angesehen. Es zeigte sich jedoch, daß wenn den zu zersetzenden Substanzen in ausreichendem Maße Hühnergülle beigemischt wird, selbst bei einer Fettsäure-oder Fettsäurerestkonzentration von 8000 mg/l noch keine wesentliche Beeinträchtigung der Gasproduktion zu verzeichnen ist; so werden bei der Zersetzung eines Gemisches aus Maschinenleimleder und Hühnergülle bei Fettsäurekonzentrationen in diesem Bereich immer noch eine unerwartet hohe spezifische Biogasproduktion von mehr als 1 m³ pro Kilogramm organische Trockensubstanz (oTS) erreicht.

Bevorzugterweise wird den zu zersetzenden Substanzen in einem solchen Maße Hühnergülle beigemischt, daß die pro Zeit verarbeitbare Menge dieser Substanzen derart gesteigert werden kann, daß innerhalb des Faulbehälters Fettsäurekonzentrationen bis zu 12.000 mg Säure pro Liter gebildet werden.

Es hat sich weiter gezeigt, daß die pro Zeit verarbeitete Menge stark fetthaltiger Substanzen, insbesondere Maschinenleimleder, derart gesteigert werden kann, daß Fettsäurekonzentrationen bis zu ca. 17.000 mg/l gebildet werden, wobei dies eine obere Grenze für die sinnvolle Verarbeitbarkeit von insbesondere Maschinenleimleder darstellen dürfte.

Da den zu zersetzenden Substanzen erfindungsgemäß ein ammoniakhaltiger Puffer, insbesondere durch Zugabe von Hühnergülle, beigegeben wird, wird bei den beiden vorstehend erwähnten, durch die Zersetzung von Maschinenleimleder und Hühnergülle gebildeten Fettsäure- bzw. Fettsäurerestkonzentration von ca. 17.000 bzw. ca. 12000 mg/l ein PH-Wert von ca. 7,6 bzw. ca. 7,9 stabilisiert. Mit diesen PH-Werten wird der den Lebensbedingungen der Methanbakterien entsprechende PH-Bereich noch nicht verlassen.

Bei der Verarbeitung von Maschinenleimleder werden bevorzugterweise von den Methanbakterien zum Aufbau von Biomasse benötigte Spurenelemente zugesetzt; diese sind in Hühnergülle in ausreichendem Maße vorhanden.

Ein weiterer, den Zersetzungsprozeß beeinflussender Parameter ist die bereits mehrfach erwähnte Ammoniakkonzentration (NH₃). In einem PH-Bereich von ca. 7,0 - ca. 7,5 werden in der Literatur maximale NH₃-Konzentrationen von 0,3 Massenprozent angegeben, im PH-Bereich von ca. 7,5 - ca. 8,0 sogar auf 0,12 Massenprozent absinkend. Theoretisch müßte damit bereits bei der Zersetzung von reiner Hühnergülle diese toxische Grenzkonzentration überschritten werden, da Hühnergülle zu 0,6 Massenprozent aus in Form von NH₃ vorliegendem Stickstoff besteht. Praktische Erfahrungen belegen jedoch, daß auch höhere Werte als 0,6 Massenprozent noch zu keiner wesentlichen Beeinflussung der Biogasbildung führen, was durch die große Menge der bei der Zersetzung stark fetthaltiger Substanzen anfallenden Säuren erklärt werden kann, da zum einen Säure durch Ammoniak, zum anderen aber auch Ammoniak durch Säure abgepuffert wird. Das Mischen von stark fetthaltigen Substanzen, insbesondere Maschinenleimleder, mit Hühnergülle führt zu einer Reduzierung der NH₃-Konzentration der Hühnergülle, da diese Substanzen kaum in Form von NH₃ vorliegenden Stickstoff aufweisen und da die bei der Zersetzung dieser Substanzen entstehenden Säuren die NH₃-Konzentration unter Bildung von NH₄⁺ erniedrigen.

Als optimaler Trockensubstanzgehalt des zu zersetzenden Substratgemisches werden 5 - 8 Massenprozent angesehen. Da Maschinenleimleder Trockensubstanzgehalte von über 20 Massenprozent aufweist, ist eine Verdünnung erforderlich. Wenn große Mengen von Maschinenleimleder pro Zeit verarbeitet werden, d. h. wenn die kontinuierliche oder diskontinuierliche Zugabe von Maschinenleimleder in den Faulbehälter und daher auch die entsprechend der Zugabe zu entnehmende Menge von Faulwasser und/oder Schlamm groß ist, ist die durchschnittliche Verweildauer des zu zersetzenden Maschinenleimleders im Faulbehälter gering, und die Zersetzung unvollständiger, d. h. die durch den Substratabbau bewirkte Reduzierung des Trockensubstanzgehalts im Faulbehälter ist entsprechend gering, weshalb bei höherem Durchsatz die zu zersetzende Substanz mit bereits relativ niedrigem Trockensubstanzgehalt dem Faulbehälter zuzuführen ist. Im Falle eines Substratgemisches aus Maschinenleimleder und Hühnergülle (die letztere hat einen Trockensubstanzgehalt von 5 - 12 Massenprozent) wird ein Trockensubstanzgehalt von ca. 10 % bevorzugt, der sich im Zuge des Substratabbaus innerhalb des Faulbehälters weiter verringert. Bei geringerem Substratdurchsatz, also bei längerer Verweilzeit des zu zersetzenden Substrats im Faulbehälter und einer entsprechend stärkeren Verringerung des Trockensubstanzgehalts infolge des Substratabbaus kann das Substrat oder Substratgemisch mit einem entsprechend höheren Trockensubstanzgehalt dem Faulbehälter zugeführt werden.

Da als optimales N/C-Verhältnis eines anaerob zu zersetzenden Substratgemisches Werte zwischen ca. 0,07 und ca. 0,1 angesehen werden, und da Maschinenleimleder, als Beispiel einer stark fetthaltigen Substanz, aber ein N/C-Verhältnis von 0,14 und die bevorzugterweise zuzusetzende Hühnergülle ein N/C-Verhältnis von 0,2 aufweisen, wird vorgeschlagen, Küchenabfälle, insbesondere Kartoffelschalen, Brot und dergleichen aber auch Weizenstroh zuzusetzen, da diese Substanzen ein N/C-Verhältnis von unter 0,05 aufweisen.

Weitere Merkmale, Vorteile und Einzelheiten der Erfindung ergeben sich aus der nachfolgenden Beschreibung des erfindungsgemäßen Verfahrens anhand einer zeichnerischen Darstellung einer zur Durchführung des Verfahrens geeigneten Biogasanlage sowie anhand einer im Zuge des Betriebs dieser Biogasanlage und der Durchführung des erfindungsgemäßen Verfahrens erstellten Tabelle.

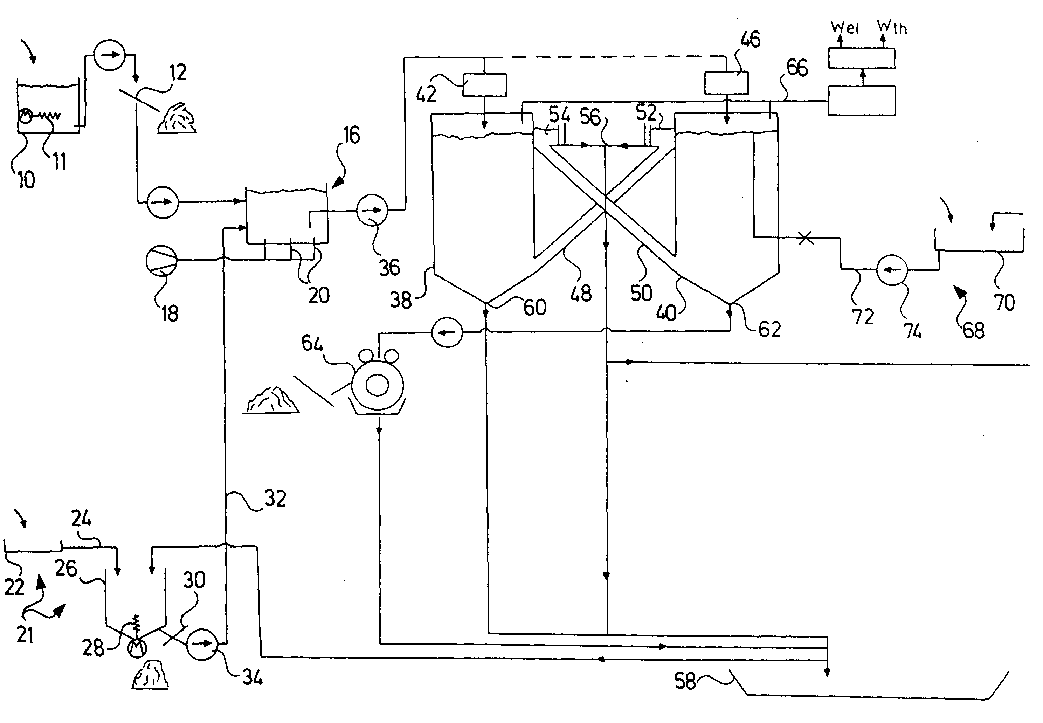

Die Zeichnung zeigt schematisch den Aufbau einer Biogasanlage, wie sie derzeit von der Anmelderin betrieben wird, und soll im folgenden als Flußdiagramm zur Beschreibung des erfindungsgemäßen Verfahrens dienen.

Die dargestellte Biogasanlage umfaßt einen Substratannahmebehälter 10 mit einer Mischvorrichtung 11 zur Aufnahme und Vermischung von Rohgülle, insbesondere Hühnergülle, Silosickersaft, Festmist sowie Fettabscheider, die unter dem Begriff grobstoffhaltiges organisches Substrat, das jedoch störstofffrei ist, zusammengefaßt werden können. Um Verstopfungen in Rohrleitungen oder in Wärmetauschern zu verhindern, werden die Grobstoffe, zu einem großen Teil mit der Hühnergülle eingebrachte Federn, durch Schwingstößelsiebe 12 abgetrennt und mit Zuschlagstoffen, wie z. B. Gartenabfällen, Erde, Häckselgut, Rindenabfällen oder dergleichen, vermischt und einer Kompostierung zugeführt. Das grobstofffreie Substratgemisch wird dann in eine Erdgrube 16 gepumpt, die auch als erste Faulstufe bezeichnet wird. In dieser ersten Faulstufe beginnt der biologische Abbau der zu zersetzenden organischen Substanzen auf fakultativ anaerobe Weise bishin zu organischen Säuren, wie insbesondere kurzkettige Fettsäuren. Die Aufenthaltsdauer des Substratgemisches in der ersten Faulstufe beträgt zwischen zwei und zehn Tagen und wird so gewählt, daß ein gezielter Voraufschluß in Abhängigkeit von dem verwendeten Substrat und von der Temperatur (Sommer, Winter) erreicht wird. Es besteht zudem die Möglichkeit, über eine mittels einer Pumpe 18 betriebene Düsenvorrichtung 20 Luft in das in der ersten Faulstufe befindliche Substratgemisch einzublasen; damit wird zum einen eine Homogenisierung des Gemisches sowie eine Sauerstoffzufuhr erreicht und zum anderen Schwefelwasserstoff, der infolge des Abbauprozesses entsteht, ausgetrieben. Letzteres führt dazu, daß der Schwefelwasserstoffgehalt des in einer nachfolgenden Verfahrensstufe erzeugten Biogases deutlich reduziert wird, was sich bei der Gasverwertung vorteilhaft auswirkt.

Für die Einbeziehung organischer Reststoffe, die nicht aus der Landwirtschaft stammen, wie z. B. Küchenabfälle, Fischabfälle, Krautreste, Biotonne, Rückstände aus der Lebensmittelindustrie, die also Störstoffe enthalten können, umfaßt die Biogasanlage eine Annahmeeinrichtung 21 für derartiges störstoffhaltiges organisches Substrat. Diese Annahmeeinrichtung 21 umfaßt eine erste Substratannahmewanne 22 sowie einen 5 m³ fassenden Stofflöser 26. Von der ersten Substratannahmewanne 22 aus wird das störstoffhaltige organische Substrat über eine Förderstrecke 24 in den Stofflöser 26 gefördert. Nach Zugabe von Gülle oder Faulwasser wird das in den Stofflöser 26 eingebrachte Substrat mittels eines Schlagwerkes 28 zerkleinert, wodurch eine Aufmaischung erreicht wird. Die Zugabe von Gülle oder Faulwasser erfolgt in einem solchen Maße, daß das entstehende pumpfähige Substratgemisch auf einen Trockensubstanzgehalt von vorzugsweise ca. 8 % gebracht wird. Dieses Substratgemisch kann dann aus dem Stofflöser 26 ausfließen, wobei mittels eines Siebes 30 im Bereich des Ablasses des Stofflösers 26 Fremdkörper, wie z. B. Metallteile, Glasscherben, Plastikreste und dergleichen, abgetrennt und anschließend deponiert werden, und wird über eine Leitung 32 mit einer Pumpe 34 der Erdgrube 16, also der ersten Faulstufe, zugeführt. Dadurch, daß in dieser ersten Faulstufe nichtlandwirtschaftliche, recht unterschiedliche organische Stoffe mit dem an erster Stelle beschriebenen Substrat gemischt werden, werden Substratunterschiede (Fette/Eiweiß/Kohlehydrate/Spurenelemente) ausgeglichen, und es kommt daher zu keinen größeren Substratschwankungen im darauffolgenden obligat anaeroben Prozeß.

Aus der ersten Faulstufe wird das vorgesäuerte Substratgemisch mittels einer Dosierpumpe 36 einem ersten und einem zweiten je 740 m³ umfassenden Faulbehälter 38, 40 zugeführt, wobei es zuvor durch Wärmetauscher 42 und 46 auf 35°C erwärmt wird. Da im zweiten Faulbehälter 40 auf noch zu erläuternde Weise große Mengen von Maschinenleimleder verarbeitet werden, wird der größte Teil des Substratgemisches (ca. 90 %) dem ersten Faulbehälter 38 zugeführt. Entsprechend der jeweiligen den Faulbehältern 38, 40 zugeführten Substratmenge wird ausgefaultes Substrat über Sedimentationsröhren 48 und 50 in Überlaufkästen 52 bzw. 54 verdrängt. Diese Sedimentationsröhren 48, 50 verlaufen von einem unteren Bereich der Faulbehälter 38 bzw. 40 in einem Winkel von etwa 45° zur Vertikalen nach oben und münden in den Überlaufkästen 52 bzw. 54. Durch den Einfluß der Schwerkraft wird das in die Überlaufkästen 52, 54 verdrängte Faulwasser von organischen Feststoffpartikeln, die innerhalb der Sedimentationsröhren 48 und 50 sedimentieren und in die Faulbehälter 38, 40 zurückrutschen, befreit, so daß in den Überlaufkästen 52, 54 eine trockensubstanzarme flüssige Phase mit einem Trockensubstanzgehalt von etwa 2 - 3 % gebildet wird. Diese flüssige Phase wird dann über ein Leitungssystem 56 in ein Speicherbecken 58 geleitet, von wo aus sie der landwirtschaftlichen Verwertung zugeführt wird, oder sie wird zur Verdünnung bei der Substratannahme verwendet.

Der biologische Abbau in den Faulbehältern 38 und 40 erfolgt auf anaerobe Weise im mesophilen Temperaturbereich zwischen 30 und 37°C, vorzugsweise bei 35°C, mittels Biogas oder Methan erzeugender anaerober Bakterien, die mit dem zu zersetzenden Substrat in die Faulbehälter eingebracht und dort vermehrt werden.

Da sich die Methanbakterien innerhalb der Faulbehälter an den organischen Feststoffpartikeln befinden, wird durch die sogenannte Biomasserückführung durch Sedimentation innerhalb der Sedimentationsröhren 48, 50 und Zurückrutschen der sedimentierten Feststoffpartikel in die Faulbehälter 38, 40 eine hohe Bakterienpopulation in dieser anaeroben oder auch methanogenen Verfahrensstufe gewährleistet. Durch die Trennung in trockensubstanzarme Phase in den Überlaufkästen 52 und 54 und in trockensubstanzreiche Phase, nämlich das in die Faulbehälter 38, 40 zurückrutschende Sediment, läßt sich auch der Feststoffgehalt innerhalb der Faulbehälter steuern; soll der Trockensubstanzgehalt in den Faulbehältern 38, 40 erhöht werden, so wird die trockensubstanzarme Phase in das Speicherbecken 58 geleitet, soll sie erniedrigt werden, so wird sie zur Verdünnung des den Faulbehältern 38, 40 zuzuführenden Substratgemisches verwendet, zusätzlich kann über Ablaßeinrichtungen 60 und 62 im Bodenbereich der Faulbehälter 38 und 40 trockensubstanzreicher Grundschlamm aus den Faulbehältern entnommen und mittels eines Feststoffseparators 64 mechanisch entwässert und als organischer Dünger verwendet werden. Die bei der Entwässerung anfallende Flüssigkeit kann z. B. der landwirtschaftlichen Verwertung zugeführt oder zur Kompostbefeuchtung oder zu Verdünnungszwecken bei der Substratannahme verwendet werden.

Die Faulbehälter 38, 40 umfassen je eine Vorrichtung zur schonenden Umwälzung des Substratgemisches sowie eine Temperiervorrichtung, die jedoch beide im Stand der Technik bekannt sind und daher keiner Beschreibung oder Darstellung bedürfen.

Das im Zuge der anaeroben Zersetzung anfallende Biogas wird kontinuierlich über geeignete Leitungssysteme 66 vom jeweils oberen Bereich der Faulbehälter 38, 40 abgezogen und anschließend zur Erzeugung thermischer oder elektrischer Energie verwendet.

Schließlich umfaßt die Biogasanlage eine zweite Substratannahmeeinrichtung 68 für störstofffreies organisches Substrat, das sich jedoch im Unterschied zu dem grobstoffhaltigen organischen Substrat, das für den Substratannahmebehälter 10 vorgesehen ist, nicht zerkleinern läßt und daher zu Versetzungen in den von dem ersten Substratannahmebehälter 10 wegführenden Rohrleitungen und in den Pumpen führen würde. Ein derartiges fremdstofffreies organisches Substrat ist Maschinenleimleder, es ist stark fetthaltig und grobstückig, jedoch aufgrund seiner Faserstruktur mechanisch kaum zerkleinerbar. Das in Containerfahrzeugen angelieferte Maschinenleimleder wird zunächst in eine zweite Annahmewanne 70 gekippt; durch Zugabe von Hühnergülle und Faulwasser aus den Überlaufkästen 52 und 54 der Faulbehälter 38 und 40 wird ein pumpfähiges Feststoff/Flüssigkeitsgemisch erzeugt, das bereits erwärmt ist, da das Faulwasser die Faulbehälter 38, 40 mit einer Temperatur von 35°C verläßt. Mit dem Faulwasser werden Spurenelemente, zum anaeroben Abbau benötigte Bakterien sowie Ammoniak zudosiert, so daß das Feststoff/Flüssigkeitsgemisch bereits annähernd die Zusammensetzung der anaeroben Phase aufweist und es somit beim Einbringen dieses Gemisches in den zweiten Faulbehälter 40 zu keinen unerwünschten Schwankungen in der Zusammensetzung des zu zersetzenden Gemisches innerhalb des Faulbehälters 40 und somit zu keinen Störungen der optimalen Prozeßbedingungen kommt. Das Feststoff/Flüssigkeitsgemisch wird über eine Rohrleitung 72 ausreichend großen Durchmessers mittels einer Rollschneckenpumpe 74 in den oberen Teil des Reaktors 40 gefördert.

Anhand der nachstehenden Tabelle werden nun verschiedene Verfahrensparameter, die in Abhängigkeit von der pro Zeit verarbeiteten Menge von Maschinenleimleder bestimmt wurden, erörtert.

| verarbeitete Masch.leimledermenge | | Gasproduktion | | CH₄-Gehalt | Fettsäure-konz. | pH-Wert |
|---|---|---|---|---|---|---|
| kg/d | kg oTS/d | m³/d | m³/kg oTS | % | mg/l | |
| 2000 | 400 | 680 | 1,7 | 72 | 1500 | 8,2 |
| 5000 | 1000 | 1400 | 1,4 | 68 | 4800 | 8,2 |
| 10000 | 2000 | 2200 | 1,1 | 65 | 7900 | 8,4 |
| 20000 | 4000 | 2500 | 0,62 | 60 | 12100 | 7,9 |
| 30000 | 6000 | 2050 | 0,34 | 50 | 17300 | 7,6 |

In der ersten Spalte von links ist die Menge des pro Tag in den Faulbehälter 40 eingebrachten Maschinenleimleders angegeben, daneben die darin jeweils enthaltene Menge organischer Trockensubstanz (oTS). In der dritten und vierten Spalte von links ist die absolute bzw. die spezifische, auf ein Kilogramm organische Trockensubstanz bezogene Gasproduktion in m³ pro Tag bzw. pro Kilogramm oTS angegeben. In der fünften Spalte ist der Methangehalt des produzierten Biogases aufgeführt. Die sechste Spalte gibt die bei den unterschiedlichen Durchsätzen sich einstellende Fettsäurekonzentration oder Fettsäurerestkonzentration an, da ja die entstehenden Fettsäuren zum größten Teil mit dem im Faulwasser enthaltenen Ammoniak reagieren, was anhand des in der letzten Spalte aufgeführten PH-Wertes des Substratgemisches im Faulbehälter 40 ersichtlich ist.

Da man aus Laborversuchen ermittelt hatte, daß beim Abbau von einem Kilogramm Maschinenleimleder in zehn Tagen mit einer Säurebildung von 95 g zu rechnen ist und da, wie eingangs bereits erwähnt wurde, als obere Grenze für die Säurekonzentration in der methanogenen Phase 2000 - 3000 mg/l angesehen wurden, gelangte man in einer groben Abschätzung unter Berücksichtigung eines Faulraumvolumens von 680 m³ zu der Auffassung, daß täglich maximal ca. 2 t Maschinenleimleder verarbeitet werden können. Wie aus der vorstehenden Tabelle jedoch ersichtlich ist, werden bei täglichen Verarbeitungsmengen von bis zu 10 t überdurchschnittliche spezifische Biogasausbeuten erzielt, die bei der Verarbeitungsmenge von 10 t pro Tag immer noch oberhalb 1 m³ Biogas pro Kilogramm oTS liegen, obschon sich im Faulbehälter 40 eine Fettsäure- oder Fettsäurerestkonzentration von 7900 mg/l einstellt. Es zeigte sich jedoch, daß durch die erfindungsgemäße Verfahrensführung selbst bei derart hohen Konzentrationen der PH-Wert nicht in den sauren Bereich abgleitet, sondern, wie sich aus der Tabelle ergibt, einen Wert von 8,4 annimmt. Selbst wenn dem Faulbehälter 40 täglich 20 oder gar 30 t Maschinenleimleder zugeführt werden und sich Fettsäurekonzentrationen von über 12.000 bzw. über 17.000 mg/l einstellen, wird der PH-Wert des Substratgemisches im Faulbehälter bei 7,9 bzw. 7,6 stabilisiert. Wenn der Durchsatz von 10 auf 20 t pro Tag gesteigert wird, führt dies nur zu einer unwesentlichen Steigerung der absoluten Gasproduktion (von 2200 auf 2500 m³/Tag) während die spezifische Gasproduktion von 1,1 auf 0,62 m³/kg oTS sinkt und der Methangehalt des Biogases von 65 auf 60 % absinkt. Bei einer Erhöhung des Durchsatzes von 20 auf 30 t pro Tag ist sogar ein Rückgang der absoluten Gasproduktion von 2500 auf 2050 m³/Tag zu verzeichnen; ein derart hoher Durchsatz ist dann sinnvoll, wenn nicht die Biogasproduktion, sondern die Verarbeitung möglichst großer Mengen Maschinenleimleders im Vordergrund steht.

Mit zunehmender Menge des pro Zeit in den Faulbehälter 40 eingebrachten Maschinenleimleders reduziert sich die Aufenthaltsdauer des Maschinenleimleders im Faulbehälter 40, da jeweils ein dem eingebrachten Volumen entsprechendes Volumen aus dem Faulbehälter 40 verdrängt wird; die Ausfaulung des Substratgemisches wird unvollständiger. Bei einem Durchsatz von mehr als 30 t pro Tag finden im Faulbehälter 40 in erster Linie Versäuerungsprozesse statt, so wie sie in der ersten Faulstufe, dort jedoch auf fakultativ anaerobe Weise, stattfinden, und die methanogene Phase müßte in einer weiteren Stufe erfolgen.

Die Möglichkeit derart hoher Verarbeitungsmengen von stark fetthaltigen Substanzen, wie insbesondere Maschinenleimleder, läßt sich auf den erfindungsgemäßen Zusatz von Faulwasser oder Gülle zurückführen. Das im Faulwasser oder in der Gülle enthaltene Ammoniak bewirkt eine Neutralisation der sich im Zuge der Zersetzung bildenden Fettsäuren und verhindert damit ein Absinken des PH-Wertes unter einen die Lebensbedingungen der Methanbakterien begrenzenden Wert.

## Patentansprüche

1. Verfahren zur Entsorgung von stark fetthaltigen Substanzen, wie insbesondere Maschinenleimleder, dadurch gekennzeichnet, daß diese Substanzen unter Beimischung eines ammoniakhaltigen, den PH-Wert stabilisierenden Puffers sowie fettspaltender und Biogas erzeugender Bakterien in einer Biogasanlage anaerob zersetzt werden.

2. Verfahren nach Anspruch 1, dadurch gekennzeichnet, daß den anaerob zu zersetzenden fetthaltigen Substanzen Hühnergülle beigemischt wird.

3. Verfahren nach Anspruch 1 oder 2, dadurch gekennzeichnet, daß den zu zersetzenden Substanzen zum Herstellen eines pumpfähigen Substratgemisches zum Einbringen in einen Faulbehälter ein flüssiges Medium beigegeben wird.

4. Verfahren nach Anspruch 3, dadurch gekennzeichnet, daß dem flüssigen Medium zuvor der Puffer sowie die fettspaltenden und Methan erzeugenden Bakterien beigegeben werden.

5. Verfahren nach Anspruch 3 oder 4, dadurch gekennzeichnet, daß als flüssiges Medium aus einem Faulbehälter entnommenes Faulwasser verwendet wird.

6. Verfahren nach einem der Ansprüche 3 - 5, dadurch gekennzeichnet, daß das flüssige Medium zumindest zum Teil aus Hühnergülle besteht.

7. Verfahren nach einem der Ansprüche 3 - 6, dadurch gekennzeichnet, daß das pumpfähige Substratgemisch in einen oberen Bereich des Faulbehälters eingebracht wird.

8. Verfahren nach einem oder mehreren der vorstehenden Ansprüche, dadurch gekennzeichnet, daß aus dem Faulbehälter entnommenes Faulwasser einer Nachfaulung in einem weiteren, im wesentlichen nur Faulwasser enthaltenden Faulbehälter unterzogen wird.

9. Verfahren nach einem oder mehreren der vorstehenden Ansprüche sowie nach Anspruch 2, dadurch gekennzeichnet, daß den zu zersetzenden Substanzen in einem solchen Maße Hühnergülle beigemischt wird, daß die pro Zeiteinheit verarbeitbare Menge dieser Substanzen derart gesteigert werden kann, daß innerhalb des Faulbehälters Fettsäurekonzentrationen bis zu 17.000 mg Säuren pro Liter gebildet werden.

10. Verfahren nach Anspruch 9, dadurch gekennzeichnet, daß Fettsäurekonzentrationen bis zu 12.000 mg Säuren pro Liter gebildet werden.

11. Verfahren nach Anspruch 9 oder 10, dadurch gekennzeichnet, daß durch den Güllezusatz innerhalb des Faulbehälters ein PH-Wert von zumindest 7,6 stabilisiert wird.

12. Verfahren nach Anspruch 11, dadurch gekennzeichnet, daß ein PH-Wert von zumindest 7,9 stabilisiert wird.

## Claims

1. Process for the disposal of substances with a high fat content such as, in particular, hide scrapings obtained using machinery, characterized in that these substances are mixed with a buffer containing ammonia and stabilizing the pH value and with fat-splitting bacteria producing a biogas and are anaerobically decomposed in a biogas plant.

2. Process as defined in claim 1, characterized in that liquid hen manure is mixed with the substances containing fat which are to be decomposed anaerobically.

3. Process as defined in claim 1 or 2, characterized in that a liquid medium is added to the substances to be decomposed in order to produce a pumpable substrate mixture for introduction into a digestion tank.

4. Process as defined in claim 3, characterized in that the buffer and the fat-splitting bacteria which produce methane are added to the liquid medium beforehand.

5. Process as defined in claim 3 or 4, characterized in that putrid water removed from a digestion tank is used as liquid medium.

6. Process as defined in any one of claims 3 to 5, characterized in that the liquid medium consists at least partly of liquid hen manure.

7. Process as defined in any one of claims 3 to 6, characterized in that the pumpable substrate mixture is introduced into an upper region of the digestion tank.

8. Process as defined in any one or several of the preceding claims, characterized in that putrid water removed from the digestion tank is subjected to a secondary putrefaction in a further digestion tank containing essentially only putrid water.

9. Process as defined in any one or several of the preceding claims and in claim 2, characterized in that liquid hen manure is added to the substances to be decomposed to such an extent that the amount of these substances treatable per time unit can be increased such that fatty acid concentrations of up to 17,000 mg acids per litre are formed inside the digestion tank.

10. Process as defined in claim 9, characterized in that fatty acid concentrations of up to 12,000 mg acids per litre are formed.

11. Process as defined in claim 9 or 10, characterized in that a pH value of at least 7.6 is stabilized inside the digestion tank by the addition of the liquid manure.

12. Process as defined in claim 11, characterized in that a pH value of at least 7.9 is stabilized.

## Revendications

1. Procédé d'élimination de substances à forte teneur en graisses, comme en particulier des raclures de peaux ou cuirs obtenues par l'usage de machines, caractérisé en ce que ces substances sont décomposées par voie anaérobique dans une installation à biogaz par admixtion d'un tampon contenant de l'ammoniac et stabilisant la valeur du pH ainsi que de bactéries effectuant une lipolyse et produisant du biogaz.

2. Procédé suivant la revendication 1, caractérisé en ce que du purin de volailles est mélangé aux substances contenant des graisses, à décomposer par voie anaérobique.

3. Procédé suivant l'une des revendications 1 et 2, caractérisé en ce qu'un milieu liquide est ajouté aux substances à décomposer pour réaliser un mélange de substrats pompable à introduire dans un réservoir de putréfaction.

4. Procédé suivant la revendication 3, caractérisé en ce qu'au milieu liquide, on ajoute tout d'abord le tampon ainsi que les bactéries effectuant une lipolyse et produisant du méthane.

5. Procédé suivant l'une des revendications 3 et 4, caractérisé en ce que, comme milieu liquide, on utilise de l'eau putride prélevée d'un réservoir de putréfaction.

6. Procédé suivant l'une des revendications 3 à 5, caractérisé en ce que le milieu liquide est constitué au moins pour une part de purin de volailles.

7. Procédé suivant l'une des revendications 3 à 6, caractérisé en ce que le mélange de substrats pompable est introduit dans une zone supérieure du réservoir de putréfaction.

8. Procédé suivant une ou plusieurs des revendications précédentes, caractérisé en ce que l'eau putride prélevée du réservoir de putréfaction est soumise à une putréfaction ultérieure dans un autre réservoir de putréfaction contenant essentiellement uniquement de l'eau putride.

9. Procédé suivant une ou plusieurs des revendications précédentes ainsi que suivant la revendication 2, caractérisé en ce qu'aux substances à décomposer, on mélange du purin de volailles en une quantité telle que la quantité traitable par unité de temps de ces substances puisse être augmentée de telle façon que des concentrations en acides gras allant jusqu'à 17.000 mg d'acides par litre soient formées à l'intérieur du réservoir de putréfaction.

10. Procédé suivant la revendication 9, caractérisé en ce que des concentrations en acides gras allant jusqu'à 12.000 mg d'acides par litre sont formées.

11. Procédé suivant l'une des revendications 9 et 10, caractérisé en ce que, par l'addition de purin, une valeur de pH d'au moins 7,6 est stabilisée à l'intérieur du réservoir de putréfaction.

12. Procédé suivant la revendication 11, caractérisé en ce qu'une valeur de pH d'au moins 7,9 est stabilisée.
